(19) Europäisches Patentamt
European Patent Office
Office européen des brevets

(11) **EP 4 321 607 A1**

(12) **EUROPEAN PATENT APPLICATION**

(43) Date of publication:
**14.02.2024 Bulletin 2024/07**

(21) Application number: **22189456.1**

(22) Date of filing: **09.08.2022**

(51) International Patent Classification (IPC):
*C12M 1/12* (2006.01)          *C12M 1/42* (2006.01)
*C12M 1/34* (2006.01)

(52) Cooperative Patent Classification (CPC):
**C12M 23/26; C12M 23/12; C12M 41/36;
C12M 41/46**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Nederlandse Organisatie voor
toegepast-natuurwetenschappelijk Onderzoek
TNO
2595 DA 's-Gravenhage (NL)**

(72) Inventors:
• **NAMELI, Alfredo**
  **2595 DA 's-Gravenhage (NL)**
• **PEETERS, Bart**
  **2595 DA 's-Gravenhage (NL)**
• **AKKERMAN, Hylke Broer**
  **2595 DA 's-Gravenhage (NL)**
• **VAN BREEMEN, Albert Jos Jan Marie**
  **2595 DA 's-Gravenhage (NL)**
• **GELINCK, Gerwin Hermanus**
  **2595 DA 's-Gravenhage (NL)**

(74) Representative: **V.O.
P.O. Box 87930
2508 DH Den Haag (NL)**

(54) **OPTICAL READING DEVICES COMPRISING CELL CULTURE UNITS WHEREIN CONTRACTILE TISSUE CAN BE CULTURED**

(57)     According to an aspect of the invention, there is provided an optical reading device comprising cell culture units wherein contractile tissue can be cultured, wherein a cell culture unit is provided with an elastic tissue support structure extending into the cell culture unit to have the contractile tissue exert a contractile force on the elastic support structure, in order to generate a deformation of the elastic support structure, the reading device further comprising one or more light generators and light sensors, wherein the elastic support structure is formed as or includes an optical element, in order to divert light from the light generators by the elastic support structure onto the one or more light sensors, so that a deformation of the elastic support structure results in the light moving over the light sensors, and a controller registering the movement of light over the light sensors, in order to measure a contractile force of the contractile tissue.

EP 4 321 607 A1

**Description**

FIELD

The present invention relates to the technical field of optical reading devices comprising cell culture units wherein contractile tissue can be cultured.

BACKGROUND

In particular, the invention relates to an optical reading device comprising a housing for receiving a test plate on which, according to a fixed pattern, cell culture units are provided wherein contractile tissue can be formed. The contractile tissue may float inside the culture units. In the prior art a cell culture unit is provided with an elastic support structure extending into the cell culture unit to have the contractile tissue exert a contractile force on the elastic support structure, in order to generate a deformation of the elastic support structure.

One of the challenges of this system is a difficulty to generate a mass scale testing environment because the system requires detailed optical study of the mechanical interaction between the tissue and the support structure, e.g. by using imaging, in particular video confocal microscopy. This is because in a typical situation, the contraction may amount to no more than several micrometers, which is difficult to study. Thus, the structure is imaged and the image needs to be studied. This kind of imaging is cost intensive and labour intensive. Current solutions therefore suffer from low throughput, provide only snap shots, and/or may interfere negatively with drugs tests.

An object of the present invention is to provide optical reading device that is easy to produce on an industrial scale, at competitive costs.

SUMMARY OF THE INVENTION

This aim and these objects, as well as other objects that will be apparent from the description below and from the accompanying drawings, are achieved, according to the invention, by a reflective structure according to claim 1, proposed below.

An optical reading device comprises cell culture units wherein contractile tissue can be cultured. The cell culture unit is provided with an elastic tissue support structure extending into the cell culture unit to have the contractile tissue exert a contractile force on the elastic support structure, in order to generate a deformation of the elastic support structure. The reading device further comprises one or more light generators and light sensors. The elastic support structure is formed as or includes an optical element, in order to divert light from the light generators by the elastic support structure onto the one or more light sensors. In this way a deformation of the elastic support structure results in the light moving over the light sensors. A controller registers the movement of light over the light sensors, in order to measure a contractile force of the contractile tissue.

Due to the invention, it is possible to provide an optical reading device wherein testing is possible without the test being disturbed by measurements. It is noted that elastic support structures of the type described above are per se known from WO2021206551. However, this is a system that is unsuitable for large area image forming.

According to the image forming system as thus described a optical reading device can be provided wherein mechanical movement can be efficiently and readily monitored on a large scale.

BRIEF DESCRIPTION OF THE DRAWINGS

Further characteristics and advantages of the elastic support structure according to the invention will be more apparent with reference to the description given below and to the accompanying figures, provided purely for explanatory and non-limiting purposes, wherein:

Figure 1 shows an embodiment of the optical reading device in exploded view, together with a schematic cell culture unit array;
Figure 2 shows alternative embodiments of elastic support structures formed optical fibers;
Figure 3 (A - E) shows an exemplary manufacturing method for creating support structures, to be combined with the array of cell culture units;
Figure 4 shows a support structure having a focusing lens;
Figure 5 shows exemplary deflection curves for an elastic pillar;
Figure 6 shows exemplary spot displacements for an elastic pillar;
Figure 7 (A - G) shows a number of support structures having optical elements.

DETAILED DESCRIPTION OF THE DRAWINGS

Hereinafter, exemplary embodiments of the present invention will be described with reference to the accompanying drawings.

Unless otherwise defined, all terms (including technical and scientific terms) used herein have the same meaning as commonly understood by one of ordinary skill in the art to which this disclosure belongs as read in the context of the description and drawings. It will be further understood that terms, such as those defined in commonly used dictionaries, should be interpreted as having a meaning that is consistent with their meaning in the context of the relevant art and will not be interpreted in an idealized or overly formal sense unless expressly so defined herein. In some instances, detailed descriptions of well-known devices and methods may be omitted so as not to obscure the description of the present systems and methods. Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be further understood that the terms "comprises" and/or "comprising" specify the presence of stated features but do not preclude the presence or addition of one or more other features. All publications, patent applications, patents, and other references mentioned herein are incorporated by reference in their entirety. In case of conflict, the present specification, including definitions, will control.

The optical reading device may be provided as a large-area optical imager either as an integrated or freestanding device to measure micro(biological) movements and derive mechanical parameters such as flow and/or contractile forces (e.g. quantitative measurements during drug screening). In one embodiment mechanically flexible micropillars act as optical fiber to guide light onto a pixel (or an array of pixels), the deflection of such optical fiber upon external stimuli translates into light intensity variations, or spot displacement. It should be noted that a high position accuracy (better than a pixel pitch) can be obtained by interpolation using both intensity and spatial information.

While example embodiments are shown for systems and methods, also alternative ways may be envisaged by those skilled in the art having the benefit of the present disclosure for achieving a similar function and result. E.g. some components may be combined or split up into one or more alternative components. Finally, these embodiments are intended to be merely illustrative of the present system and should not be construed as limiting the appended claims to any particular embodiment or group of embodiments. Thus, while the present system has been described in particular detail with reference to specific exemplary embodiments thereof, it should also be appreciated that numerous modifications and alternative embodiments may be devised by those having ordinary skill in the art without departing from the scope of the present systems and methods as set forth in the claims that follow. The specification and drawings are accordingly to be regarded in an illustrative manner and are not intended to limit the scope of the appended claims.

Preferably, the optical reading device has a size which is only slightly larger than a standard culture plate, so that the reading device can be included and read in an incubator. As a result, it becomes possible to perform real-time measurements, without the conditions under which a cell culture takes place being altered. Due to such a configuration, the reader can be designed compactly which can be coupled in a planar manner, so that this can be placed as a whole into the incubator.

While different light guiding structures may be contemplated, a convenient form that combines the elastic structure and the light guide is provided by an optical element designed as a light fiber. The optical element may be a refractive or reflective element, so that light is refracted or reflected to form a light beam directed onto the light sensors. This enlarges the physical deflection of the structure somewhat, so that it can be measured more easily. The elastic support structure may be provided on a support plate separate from the test plate and to be stacked thereon, so that the elastic support structures protrude into the cell culture units of the test plate. Having a separate support plate is a convenient way of providing an optically optimized structure, that is yet suitable for the contractile tissue to be grown on, and arranging an optical structure inside the culture unit. For example, the support plate may be additionally provided with through holes for receiving fluids from a well plate, in order to provide the cell culture units with culturing fluids. Also, light generators may be provided on the support plate. Furthermore, the support plate may be provided with light fibers provided in a pattern corresponding to the cell culture units. Alternatively, the support plate is provided with a light shielding layer on a support plate face to only allow light through the light fibers. For example, the optical element may be a light shielding element provided in the elastic support structure, so that a light is at least partially shielded by the light shielding element to result in a sensed registration of moving light. In a further embodiment, the housing comprises a base plate, in which light sensors are provided, which base plate has a shape such that it can be coupled to the test plate for providing optical contact. In a further example the light sensors are pixelated. In some embodiments the optical elements form a sub-pixel size light beam, so that a moving spot modulates a single pixel signal. This provides a very fast read out of small deviations, made possible without much processing load, and therefore suitable for further efficient scaling. In an embodiment the light sensors are formed by a pixelated organic photodetector. The elastic support structure may further be provided with an electrical conductor for providing an electrode to stimulate the contractile tissue.

In one embodiment the elastic support structures may be spaced apart by a distance of between 0.1 and 10 mm, preferably between 0.1 and 5 mm, preferably between 0.1 and 3 mm, preferably between 0.1 and 2 mm, preferably between 0.1 and 1 mm, for example about 0.6 mm or about 0.9 mm.

With further reference to Figure 1 an embodiment is shown of the optical reading device 1 in exploded view, together with a schematic cell culture unit array 6. The optical reader 1 comprises a housing 2 having a planar test plate 3, on which cell culture units 6 are provided. The cell culture units 6 of the test plate 3 form a fixed pattern, in the example of the drawing a matrix-shaped pattern, of a number of wells which is common in practice, such as, for instance, a 96-well plate. In the drawing, for clarity's sake, only a limited number of wells are represented. On a further sensor part 7 optical conversion elements 4 are, for instance, photodiodes, or a different type of semi-conductors which are sensitive to a relevant optical range.

The conversion elements 4 have been provided in the sensor part 7 in a similar type of pattern to the wells 6 of the test plate 3. The device according to the invention has a size which virtually corresponds to that of a standard test plate, so that the reading device can be included in an incubator (not shown) for culturing biological material and can be read in situ, without mechanical disturbances occurring, in particular, without the plate having to be taken from the incubator. To that end, the invention comprises a method for testing contractile tissue with an optical reader, the method comprising the steps of providing the contractile tissue in a test plate 3 including the cell culture units 6, coupling the reader to the test plate and inserting the reader into an incubator, while the measuring signals coming from the reader are stored in a memory of the reader and/or are outputted to a central processing unit.

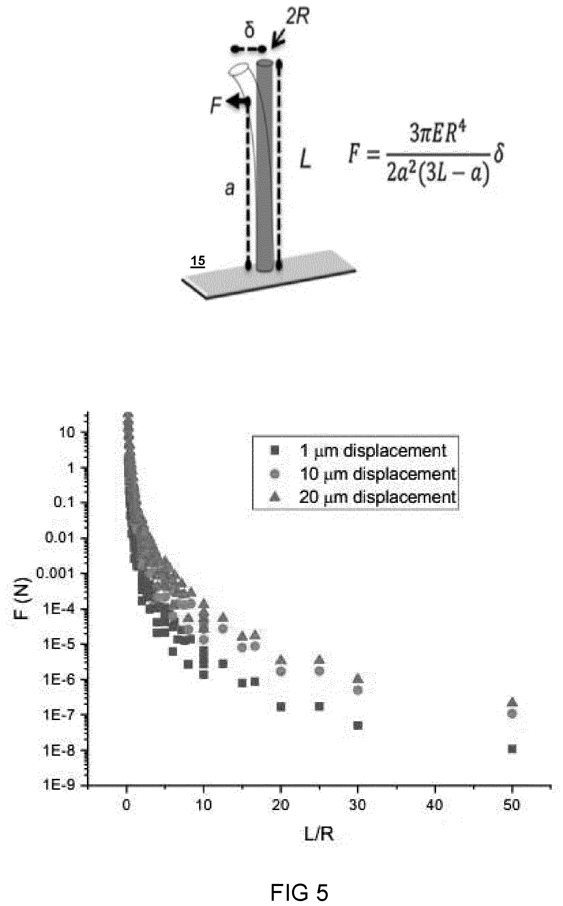

On the test plate 3, coupling elements 8 may be present in the form of a groove, in which the test plate can be slid and fixed for forming a fixed connection with the optical reader 1. Furthermore, elastic support structures 15 are provided on a support plate 5 separate from the test plate 3 including the cell culture units 6 and to be stacked thereon. In this way the elastic support structures 15 protrude into the cell culture units 6 of the test plate 3. The elastic support structures 15 are suitable for supporting contractile tissue so that the tissue is able to exert a contractile force on the support structure, to deflect it, as described here below. The support structure 15 to this end includes an optical element that may have different embodiments as illustrated in Figure 5. In the shown embodiment the optical element is designed as a light fiber that is suited for providing a support for the tissue, e.g. by having a biocompatible cladding. Accordingly the support plate 5 is provided with light fibers 15 provided in a pattern corresponding to the cell culture units 6.

By means of connections 9, optical converters (e.g. formed by pixelated organic photodetectors) 4 are connected to an electronic control unit 10, for controlling the respective converters 4, in particular for controlling the timing of the reading of the signals from a converter. With the aid of an external coupling, the control unit 10 can be connected to a computer as peripheral equipment.

The reader 1 is suited for analyzing light coming from wells 6 in the test plate 3. To that end, in the optical reader, 1 light generators 13 are provided on the support plate 5 but may also be coupled via other light coupling structures. In the shown embodiment, the light source is part support plate 5 comprises one or more light generators and light sensors, in addition to elastic support structures 15 formed as an optical element, in order to divert light from the light generators 13 by the elastic support structure onto light sensors of the sensor part 7. The reader may further comprise additional structure, e.g. a well plate 11. In the shown structure support plate 5 is additionally provided with through holes for receiving fluids from the well plate, in order to provide the cell culture units with culturing fluids.

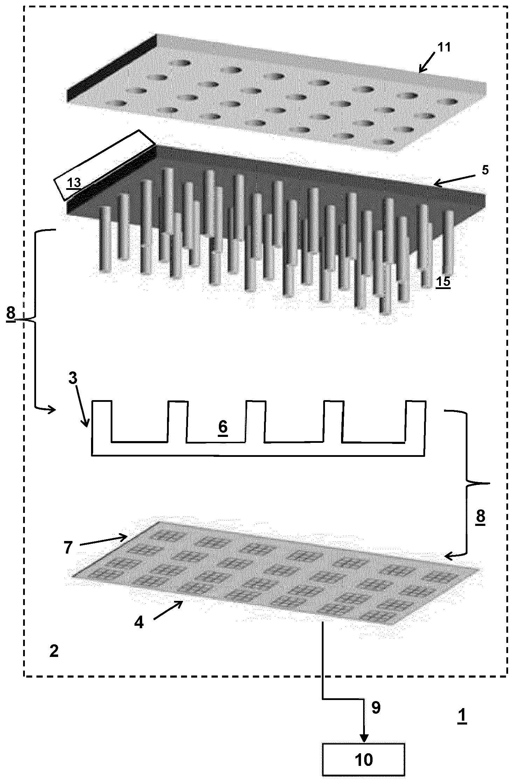

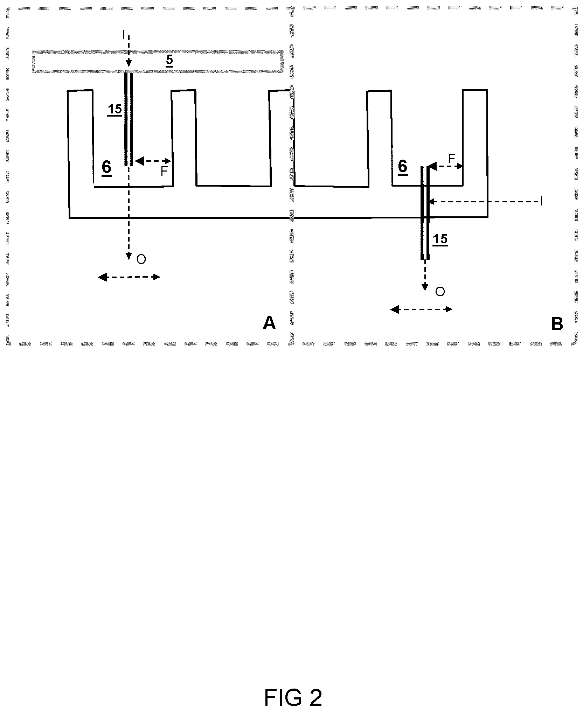

Figure 2 shows alternative embodiments of elastic support structures formed as optical fibers. In the variant A fiber 15 is part of a support plate 5 or other support structure. Tissue attached to the fiber 15 exerts a contractile force F to it, e.g. by having an attachment to a part of the well or other support element (not shown). Light I is injected into the fiber, resulting in a moving beam O that can be detected. In Variant B fiber may be part of the cell culture unit 6, e.g. by protruding through a bottom wall. Contractile force F results in a moving fiber, into which light is injected at I. The moving fiber 15 will similarly result in a moving beam at O.

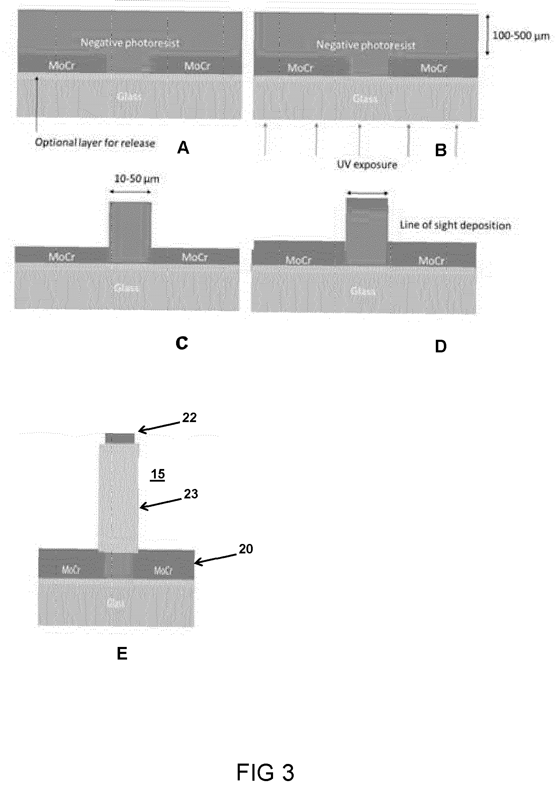

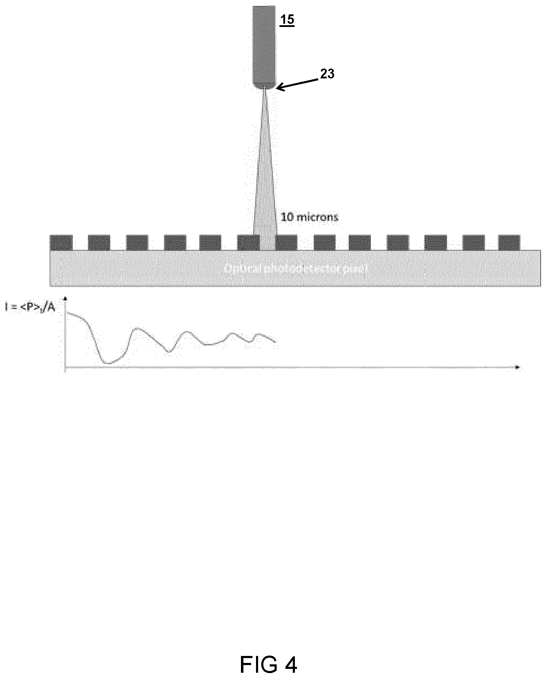

Figure 3 shows an exemplary manufacturing method for creating support structures, to be combined with the array of cell culture units. The fibers are typically very small and very thin and can be placed at relative short distances, e.g. between 0.1 and 10 mm. The fiber length is typically in a range of 0.1 and 0.5 mm, whereas the diameter is even a factor 10-15 smaller, for R/L (see Figure 4) ranging between 8 and 15.

In the illustrated example the fiber diameter ranges between 10 and 50 micrometer. The fibers 15 are produced by first selectively etching a shielding layer 20 provided on a glass plate, or on a transparent release layer. The layer thickness may be anywhere between 1 and 100 micron, so that a pattern of corresponding through holes can be etched. In the example the shielding layer is a MoCr layer, but other suitable layers are known to the skilled person. Accordingly, the support plate is provided with a light shielding layer to only allow light through the light fibers. A fluid transparent resin is provided on the shielding layer of a suitable thickness corresponding to the fiber length. The whole structure (except the shielding layer 20) is optically transparent and the top of the pillars 15 can be made reflective by adding a suitable top layer 22 on the fiber (see Figure D), such that a reflected signal from the top of the pillars can be detected in reflection. In this configuration, preferably a collimated light at an angle can lead to better accuracy. Another advantage is that with this configuration the light source and the optical readout can be placed on the top or at the bottom of the pillar structure. Eventually (Figure E) a cladded fiber is produced, by cladding it with a low - refractive index layer (relative to the fiber core), in order to provide optimal light guiding in the fiber. An additional biocompatible layer may be added on top, depending on the nature of the low-refractive index layer. Alternatively, the optical element 15 may include a light shielding element provided in the elastic support structure, so that a light is at least partially shielded by the light shielding element to result in a sensed registration of moving light.

Alternative to a reflective top layer a refractive optical element may be provided, e.g. a focusing lens of a type described in Henry E. Williams, et al., Fabrication of three-dimensional micro-photonic structures on the tip of optical fibers using SU-8, Optics Express Vol. 19, Issue 23, pp. 22910-22922, (2011)), see Figure 3. In this way, a refractive element 23 can be provided, so that light is refracted to form a light beam directed onto the light sensors. Spot sizes even smaller than a typical pixel size can be obtained; for instance, assuming a spot size of 10 microns, one can easily track the light intensity signal, using an array of light-absorbing (or reflective) material that is patterned on top of an OPD. The linewidth and the spacing of such absorbers can be the same size of the light spot diameter (e.g. 10 microns). A thin layer (in the order of 100 of nm) can already serve the purpose.

As the optical fiber is bent due to the force applied force by a tissue (or by a flow), the light spot is moved and partially hits an absorbing pattern as disclosed further below. This effectively modulates the detected intensity, rendering possible to detect small displacements at a very high read-out speed, since only one pixel per device is needed.

Figure 5 shows exemplary deflection curves for an elastic pillar 15, e.g..of a fiber structure as previously disclosed. In another embodiment a hollow-pillar can be realized a reflective layer is intentionally left exposed in the center of the pillar. This can act as a mirror when backlight illumination is used so that light is then reflected back onto the OPD thereby increasing the sensitivity. Looking at the hollow pillar as a collimator the use of a mirror at one side will make the collimator appear e.g. twice as long increasing the aspect ratio with a factor of 2, depending on the pillar dimensions. A similar deflection of the pillar will result in a larger change in signal amplitude.

$$ F = \frac{3\pi E R^4}{2a^2(3L - a)} \delta $$

Where E is the Young's modulus, R the radius of the circular pillar, L the height of the pillar, the point where the cells/tissues are attached and $\delta$ the displacement. Suitable pillar materials may be e.g. PDMS, TMMF S2000 & TMMR S2000, or PMMA having Young's moduli ranging between 0.57 - 3.7 MPa (PDMS); between 2.1 -3.8 GPa (TMMF/TMMR); and 2.9 GPa (PMMA).

A corresponding Force/displacement diagram for different L/R values for the case of 0.57 MPa Young's modulus and for different displacements is shown e.g. a displacement of 20 micron for forces as low as 1E-6 Newton and L/R value of about 20-50 is possible for a PDMS pillar.

The above calculations relate the displacement to the force exerted on the pillar of different aspect ratio. If forces of 10-6 N are to be measured, one should be able to optically detect displacements of 1 micron, for which aspect ratio of ~15 are sufficient or alternatively displacements of 10 microns, for which aspect ratio of 30 are required.

Typical force values range in the order from 10-12 N to 1 N single cells tissues.

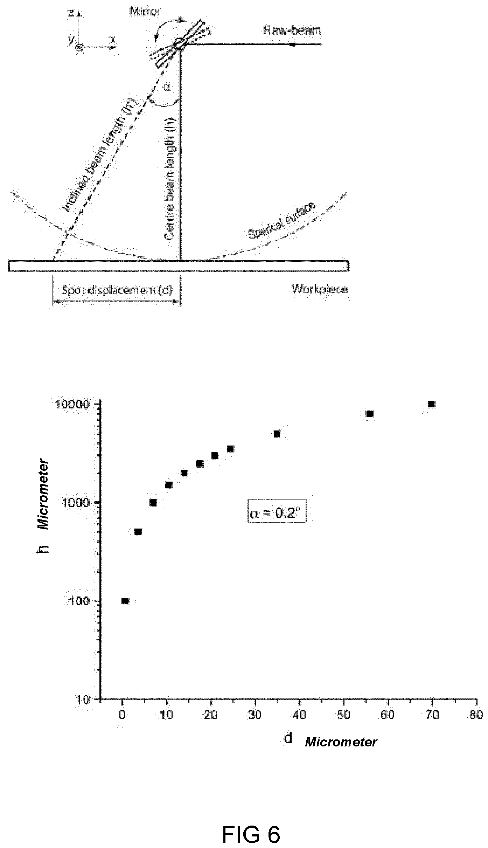

Figure 6 shows exemplary spot displacements for an elastic pillar. Additionally, an estimation of the 'amplification' provided by the micromirror configuration is provided below. Assuming a tilting of 0.2 degrees for the micromirror upon cell/tissue exerted force, one can calculate the light spot displacement, $d$, projected on an OPD array positioned at a distance $h$. For example, a displacement (d) can be approximated by tan 2θ = d/h, where h is the distance between the mirror and the detector. A small angle of 0.2° might lead to a measurable spot displacement enabling. For pixel sizes of ~100 $\mu$m, a 10 $\mu$m displacement is expected to be measurable with enough signal to noise. Hence with the configuration of Fig 3, for example, assuming a displacement of 1 micron can cause a tilt of 0.2° forces down to 10-100 nN can become accessible by projecting the light onto a 1000 micron distant OPD array.

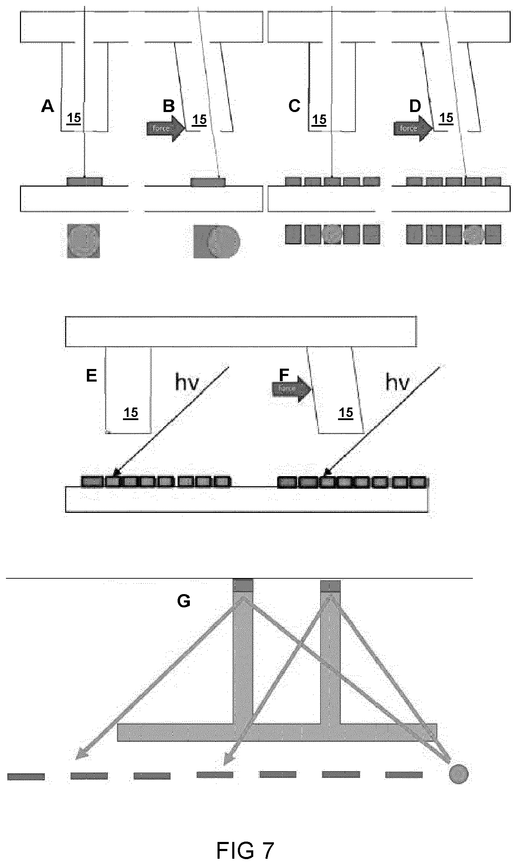

Figure 7 shows a number of support structures having optical elements, wherein a deformation of the elastic support structure results in the light moving over the light sensors in order to register a contractile force of the contractile tissue.

In Figure 7A no force is exerted on the micro pillar, which results in a light spot (circle) being centered at known position (square). When a lateral force is applied the light through the micropillar leaves the light under an angle (Figure 7B). In Figure 7C and 7D such micromechanical movement can be detected with high precision (down to 5 microns) by having light under an angle and casting the shadow of a pillar structure onto a pixel array. In a corresponding way, in Figure 7E light shadowing may be used to have light moving over the light sensors in order to register a contractile force of the contractile tissue. The micropillar 15 may thus also reflect or absorb the light which results in varying pixels that are not illuminated (Figure 7F). By having illumination under a shallow angle, micromovements of hanging structures are 'amplified' over the shadow/light projection.

In Figure 7G the whole structure is optically transparent and the top of the pillars is reflective, such that a reflected signal from the top of the pillars can be detected in reflection. In this configuration, preferably a collimated light at an angle can lead to better accuracy. Another advantage is that with this configuration the light source and the optical readout can be placed on the top or at the bottom of the pillar structure.

In the presence of the contractile tissue exerting a force on the elastic support structures light deflections can be registered by sensor 4 on sensor plate 7.

With the optical reader it is possible, in a non-invasive manner, to perform measurements on biological material, for instance, so that its vitality can be analysed in vitro. It provides high-throughput and parallelized images (e.g. acquisition of complete well-plate with highframe rate) without recurring to bulky instrumentation. It can be integrated to well-plate formats or conceived as a free-standing platform for optical monitoring of micro(bio)mechanical movements. Using light no current/voltages are in direct contact with biological sample, limiting the risk of influencing the measurement. It enables parallel recording/ measuring of micro-mechanical movements from multiple-organs or same interacting with different drugs over long time (days) with high fps. The solution can be miniaturized so the well-plate can remain in the incubator (there is no need for off-line measurements, that potentially are disturbing and lower the quality of cell cultures). Besides a generalized high resolution imager applicable to all kinds of measurement configurations, the imaging plate can be customized in design to match a designated measurement setup. For example, by limiting high resolution arrays of photosensitive pixels only close to the center location of the wells in a smart well plate, the total number of pixels within the array can be reduced by a large factor and the frame rate can increase with the same factor since it is determined by the speed of the readout IC and number of lines in the imager. Furthermore, the photosensitive pixel area can be made smaller than the pixel pitch. This allows for measurements in reflection mode.

Since in the case of a test plate the recording of such micromovements is needed only in pre-determined zones of a large-area device, a series of structures is disclosed with the aim of enhancing small movements detection with a limited number of pixels that are present in the above mentioned pre-determined areas. In this way high fps is achievable as well as high-resolution over large area.

Although the invention has been elucidated on the basis of the preferred embodiment, it is also possible to use different embodiments, which also fall within the scope of the claims. For instance, light conductors can then be used, which conduct the light of the light-receiving areas to a processing unit which can even be disposed outside an incubator, or otherwise.

Such variations are understood to fall within the scope of the invention as defined in the following claims.

**Claims**

1. An optical reading device comprising cell culture units wherein contractile tissue can be cultured, wherein a cell culture unit is provided with an elastic tissue support structure extending into the cell culture unit to have the contractile tissue exert a contractile force on the elastic support structure, in order to generate a deformation of the elastic support structure, the reading device further comprising one or more light generators and light sensors, wherein the elastic support structure is formed as or includes an optical element, in order to divert light from the light generators by the elastic support structure onto the one or more light sensors, so that a deformation of the elastic support structure results in the light moving over the light sensors, and a controller registering the movement of light over the light sensors, in order to measure a contractile force of the contractile tissue.

2. An optical reading device according to any preceding claim, **characterized in that** the optical element is designed as a light fiber.

3. An optical reading device according to any preceding claim, **characterized in that** the optical element is a refractive or reflective element, and that light is refracted or reflected to form a light beam directed onto the light sensors.

4. An optical reading device according to any preceding claim, wherein the elastic support structure is provided on a support plate separate from a test plate including the cell culture units and to be stacked thereon, so that the elastic support structures protrude into the cell culture units of the test plate.

5. The optical reading device according to claim 4, wherein the support plate is provided with through holes for receiving fluids from a well plate, in order to provide the cell culture units with culturing fluids.

6. The optical reading device according to claim 4 or 5, wherein the light generators are provided on the support plate.

7. The optical reading device according to any claim 4 - 6, wherein the support plate is provided with light fibers that are extended outside of the cell culturing unit.

8. The optical reading device according to claim 7, wherein the support plate is provided with a light shielding layer on a support plate face to block ambient light.

9. An optical reading device according to any preceding claim, **characterized in that** the optical element comprises a light shielding element provided in the elastic support structure, and that a light is at least partially shielded by the light shielding element to result in a sensed registration of moving light.

10. An optical reading device according to any preceding claim, **characterized in that** the housing comprises a base plate, in which light sensors are provided, which base plate has a shape such that it can be coupled to a test plate including the cell culture units for providing optical contact.

11. An optical reading device according to any one of the preceding claims, wherein the light sensors are pixelated.

12. An optical reading device according to claim 11, wherein the optical elements form a sub-pixel size light beam, so that a moving spot modulates a single pixel signal.

13. An optical reading device according to any preceding claim, wherein the light sensors are formed by a pixelated organic photodetector.

14. An optical reading device according to any preceding claim, wherein the elastic support structure is provided with an electrical conductor for providing an electrode to stimulate the contractile tissue.

FIG 2

A — Negative photoresist / MoCr / MoCr / Glass / Optional layer for release

B — Negative photoresist / MoCr / MoCr / Glass / 100-500 µm / UV exposure

C — 10-50 µm / MoCr / MoCr / Glass

D — MoCr / MoCr / Glass / Line of sight deposition

E — 22 / 15 / 23 / MoCr / MoCr / Glass / 20

FIG 3

FIG 4

$$F = \frac{3\pi E R^4}{2a^2(3L-a)}\delta$$

FIG 5

FIG 6

FIG 7

# EUROPEAN SEARCH REPORT

**Application Number**

EP 22 18 9456

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | OSAKI TATSUYA ET AL: "On-chip 3D neuromuscular model for drug screening and precision medicine in neuromuscular disease", NATURE PROTOCOLS, NATURE PUBLISHING GROUP, GB, vol. 15, no. 2, 13 January 2020 (2020-01-13), pages 421-449, XP037005236, ISSN: 1754-2189, DOI: 10.1038/S41596-019-0248-1 [retrieved on 2020-01-13] * the whole document * | 1 | INV. C12M1/12 C12M1/42 C12M1/34 |
| A | WO 2021/158598 A1 (TARA BIOSYSTEMS INC [US]) 12 August 2021 (2021-08-12) * paragraph [00105] – paragraph [00106] * * paragraph [0042] – paragraph [0046] * * paragraph [00204] * * paragraph [00226] * * claims * | 1 | |
| A | WO 2018/140497 A1 (EFIMOV IGOR R [US]; QIAO YUN [US] ET AL.) 2 August 2018 (2018-08-02) * paragraph [0091] * * paragraph [0111] * * paragraph [0128] * * figures * | 1 | **TECHNICAL FIELDS SEARCHED (IPC)** C12M |
| A,D | WO 2021/206551 A1 (RIVER BIOMEDICS B V [NL]) 14 October 2021 (2021-10-14) * the whole document * | 1 | |

The present search report has been drawn up for all claims

| Place of search | Date of completion of the search | Examiner |
|---|---|---|
| Berlin | 27 January 2023 | Panzica, Gian Paolo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 18 9456

27-01-2023

| Patent document cited in search report | | Publication date | Patent family member(s) | | Publication date |
|---|---|---|---|---|---|
| WO 2021158598 | A1 | 12-08-2021 | NONE | | |
| WO 2018140497 | A1 | 02-08-2018 | US | 2019376014 A1 | 12-12-2019 |
| | | | WO | 2018140497 A1 | 02-08-2018 |
| WO 2021206551 | A1 | 14-10-2021 | NONE | | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021206551 A

**Non-patent literature cited in the description**

- **HENRY E. WILLIAMS et al.** Fabrication of three-dimensional micro-photonic structures on the tip of optical fibers using SU-8. *Optics Express,* 2011, vol. 19 (23), 22910-22922